(19) Europäisches Patentamt European Patent Office Office européen des brevets

(11) **EP 4 052 645 B1**

(12) **EUROPEAN PATENT SPECIFICATION**

(45) Date of publication and mention of the grant of the patent:
**29.01.2025   Bulletin 2025/05**

(21) Application number: **22159104.3**

(22) Date of filing: **28.02.2022**

(51) International Patent Classification (IPC):
*A61B 5/0537* (2021.01)      *A61B 5/107* (2006.01)

(52) Cooperative Patent Classification (CPC):
**A61B 5/4509; A61B 5/0537; A61B 5/1072;**
**A61B 5/6825; A61B 5/6829;** A61B 5/4566

(54) **METHOD OF BIOIMPEDANCE TECHNOLOGY TO EVALUATE LOCAL OR WHOLE BODY BONE MINERAL DENSITY**

VERFAHREN ZUR BIOIMPEDANZTECHNIK ZUR BEWERTUNG LOKALER ODER VOLLSTÄNDIGER KNOCHENMINERALDICHTE

PROCÉDÉ DE TECHNOLOGIE DE BIOIMPÉDANCE POUR ÉVALUER LA DENSITÉ MINÉRALE OSSEUSE LOCALE OU DU CORPS ENTIER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **05.03.2021   TW 110107862**

(43) Date of publication of application:
**07.09.2022   Bulletin 2022/36**

(73) Proprietor: **Starbia Meditek Co., Ltd.**
**Taichung City (TW)**

(72) Inventor: **Hsieh, Kuen-Chang**
**Taichung City (TW)**

(74) Representative: **Straus, Alexander**
**2K Patent- und Rechtsanwälte**
**Bajuwarenring 14**
**82041 Oberhaching (DE)**

(56) References cited:
**KR-A- 20200 023 915     US-A1- 2002 156 378**

• **ANTUNES MELISSA ET AL: "Total and regional bone mineral density are associated with cellular health in older men and women", ARCHIVES OF GERONTOLOGY AND GERIATRICS, ELSEVIER, AMSTERDAM, NL, vol. 90, 15 June 2020 (2020-06-15), XP086246960, ISSN: 0167-4943, [retrieved on 20200615], DOI: 10.1016/ J.ARCHGER.2020.104156**

EP 4 052 645 B1

**Description**

**BACKGROUND OF THE INVENTION**

**1. Field of the Invention**

The present invention is related to bone mineral density evaluation technology, and particularly refers to a method of bioimpedance technology to evaluate local or whole body bone mineral density.

**2. Description of the Related Art**

Most of the current bone mass inspection methods are measured according to the different absorption levels of ionized radiation by bone and soft tissue. Among them, the use of dual energy X-ray absorptiometry (DXA) to express bone mineral density (BMD) by mineral mass (g/cm$^2$) is widely accepted.

However, although the method of applying DXA to measuring BMD is accepted by everyone, because DXA measurement is expensive, and its measurement location must be implemented in a hospital or related professional institution, it cannot be applied to home health care.

In addition, each measurement of DXA takes tens of minutes. Therefore, the current method of measuring bone mineral density using DXA is costly and time-consuming, and it is not convenient to use.

A method of bone mineral density evaluation according to the state of the art is for instance described in KR20200023915A.

**SUMMARY OF THE INVENTION**

The present invention has been accomplished under the circumstances in view. It is the main object of the present invention to provide a method of bioimpedance technology to evaluate local or whole body bone mineral density, which, compared with the prior art, is low in cost, fast, and convenient in use.

In order to achieve the above-mentioned purpose, a method of bioimpedance technology to evaluate local or whole body bone mineral density provided by the present invention comprises the following steps:

Step (a) Measure the resistance (R) and reactance (Xc) of a subject with a bioimpedance measuring instrument, and obtain the height of the subject.

Step (b) The bone mineral density of the subject's local or whole body is calculated by the bioimpedance measurement instrument by the following calculation formula:

$$BMD = a + b\,R/H + d\,Xc/H,$$

where a, b, and d are weighting coefficients; R is the resistance of the subject; Xc is the reactance of the subject; H is the height of the subject.

Thereby, a method of bioimpedance technology to evaluate local or whole body bone mineral density provided by the present invention only uses a bioimpedance measuring instrument to obtain the resistance, reactance, and height of the subject, and then calculates the formula BMD=a+b R /H+d Xc/H to obtain the bone mineral density. Compared with the prior art, the present invention is low in cost, fast, and convenient in use.

**BRIEF DESCRIPTION OF THE DRAWINGS**

FIG. 1 is a flowchart of a preferred embodiment of the present invention.
FIG. 2 is a block diagram of a preferred embodiment of the present invention.
FIG. 3 is a GLM regression analysis diagram of a preferred embodiment of the present invention.

**DETAILED DESCRIPTION OF THE** INVENTION

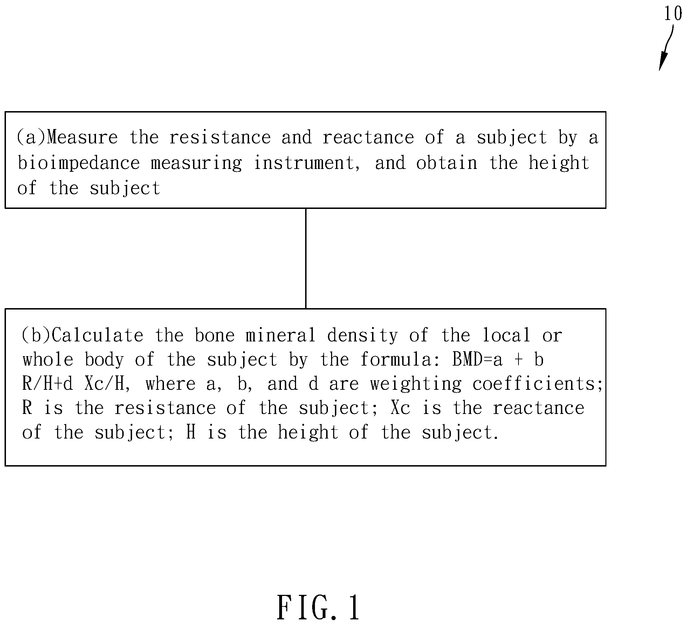

In order to explain the technical features of the present invention in detail, the following is a preferred embodiment, and the descriptions are as follows in conjunction with FIGS. 1-3. The method of bioimpedance technology to evaluate local or whole body bone mineral density **10** provided by the present invention comprises the steps of (FIG. 1):
Step (a) Use a bioimpedance measuring instrument **20** to measure the resistance and reactance of a subject, and obtain the height of the subject.

In this preferred embodiment, the height of the subject is measured by a height rod and manually inputted into the bioimpedance measuring instrument **20,** and the accuracy of the height rod is set to 0.5 cm (but not limited to this). Thereby, the height that best meets the current situation of the subject can be obtained, so as to improve the accuracy of the evaluation result of the present invention. In other preferred embodiments, the subject's height can also be directly provided by the subject, and transmitted to the bioimpedance measuring instrument **20** in an automatic and wired/wireless manner. Therefore, the selection of the bioimpedance measuring instrument **20** and the way the bioimpedance measuring instrument **20** obtains the height of the subject are not limited to the present preferred embodiment.

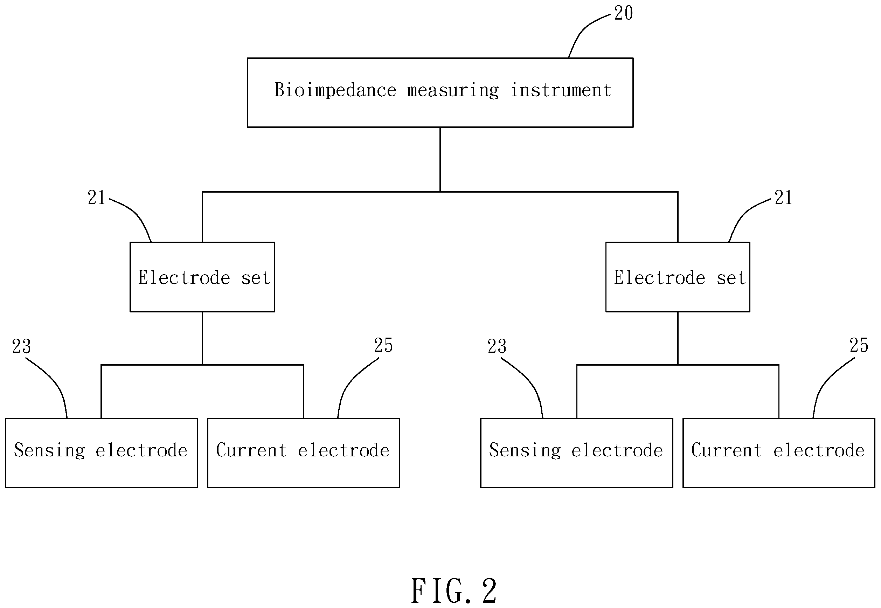

As shown in Figure 2, in the preferred embodiment, the bioimpedance measuring instrument **20** measures the resistance and reactance of the subject, and the subject performs the measurement of the bioimpedance measuring instrument **20** in a supine position, thereby reducing evaluation error. For the plural electrode sets **21** of the bioimpedance measuring instrument **20,** the number of the plural electrode sets **21** is two as an example. The plural electrode sets **21** contact the subject's right hand and right foot to form a measurement loop. Each electrode set **21** comprises a sensing electrode **23** and a current electrode **25**. The sensing electrode **23** and the current electrode **25** are separated by 5 cm, based on the characteristics of bioimpedance measurement, thereby improving the accuracy of the evaluation of the present invention. In other preferred embodiments, the subject can also perform the measurement of the bioimpedance measuring instrument **20** in a standing position. The plural electrode sets **21** can also be measured by contacting the subject's hands, feet, right hand to left foot, left hand to right foot, left hand to left foot, etc. Under different measurement modes, the number of the plural electrode sets **21** can also exceed two. The sensing electrode **23** and the current electrode **25** can also be between 1-5 cm or 5-10 cm. Therefore, the number of the plural electrode sets 21 and the manner in which the subject conducts the resistance and reactance measured by the bioimpedance measuring instrument **20** are not limited to this preferred embodiment.

In the present preferred embodiment, the bioimpedance measuring instrument **20** measures the body mineral density of the subject's body ($BMD_{total}$), lumbar spine ($BMD_{LS}$) and right upper limb ($BMD_{right\ arm}$), a total of three parts of bone mineral density. In other preferred embodiments, it is also possible to choose to measure other parts of the subject according to needs, and to increase or decrease the measured parts of the subject according to needs.

Step (b) The bone mineral density of the subject's local or whole body is calculated by the following formula:

$$BMD = a + b\ R/H + d\ Xc/H,$$

where a, b, and d are weighting coefficients; R is the resistance of the subject; Xc is the reactance of the subject; H is the height of the subject.

In the present preferred embodiment, the weighting coefficients of a, b, and d are obtained by using DXA for the whole body, lumbar spine, and right upper limb of hundreds of testers, after obtaining the average bone mineral density of the three positions. In other preferred embodiments, the weighting coefficients of a, b, and d can also be obtained after measurement by a quantitative ultrasonic inspection instrument, quantitative computed tomography, or traditional X-ray inspection. And, other parts of the tester can be selected to be measured according to the needs, and the tester's measurement parts can be increased or decreased according to the needs. Therefore, the instrument and the body parts of the tester are not limited to the present preferred embodiment.

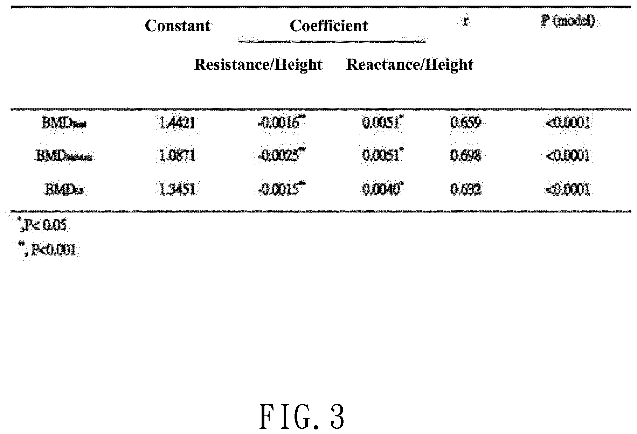

As shown in FIG. 3, it is the result of the present preferred embodiment. In the GLM regression analysis, the corresponding weighting coefficients and correlations show that the R/H and Xc/H values of the subject are positively correlated with the values of the whole body, lumbar spine and right upper limb measured by DXA.

It can be proved that the method of bioimpedance technology to evaluate local or whole body bone mineral density provided by the present invention only uses the bioimpedance measuring instrument to obtain the resistance, reactance, and height of the subject, and the bone mineral density can be measured after calculating with the formula BMD=a+b R/H+d Xc/H. Compared with the prior art, the present invention is low in cost, fast, and convenient in use.

**Claims**

1. A method of bioimpedance technology to evaluate local or whole body bone mineral density (10), comprising the steps of:

   (a) measuring a resistance and a reactance of a subject by a bioimpedance measuring instrument (20), and obtaining a height of said subject;
   (b) calculating the bone mineral density of the local or whole body of said subject by the bioimpedance measuring instrument (20) by the following formula:

   $$BMD = a + b\ R/H + d\ Xc/H,$$

   where a, b, and d are weighting coefficients; R is the resistance of said subject; Xc is the reactance of said subject; H is the height of said subject.

2. The method of bioimpedance technology to evaluate local or whole body bone mineral density (10) as claimed in claim 1, wherein in step (a), the method for said bioimpedance measuring instrument (20) to measure the resistance and reactance of said subject is to measure plural electrode sets (21) of said bioimpedance measuring instrument (20), said plural electrode sets (21) contacting the hands and feet, hands or feet of said subject to form a measurement loop, each said electrode set comprising a sensing electrode (23) and a current electrode (25), said sensing electrode (23) and said current electrode (25) being 1-10 cm apart.

3. The method of bioimpedance technology to evaluate local or whole body bone mineral density (10) as claimed in claim 1, wherein in step (a), said subject is to perform the measurement of said bioimpedance measuring instrument (20) in a supine, standing or

**Patentansprüche**

1. Bioimpedanz-Technologie-Verfahren zur Bewertung der lokalen oder Gesamt-Körper-Knochenmineraldichte (10), welches die Schritte umfasst:

   (a) Bestimmen eines Widerstands und einer Reaktanz einer Person durch ein Bioimpedanz-Messgerät (20) und Ermitteln einer Größe der Person;
   (b) Berechnung der Knochenmineraldichte des lokalen oder gesamten Körpers der Person durch das Bioimpedanzmessgerät (20) gemäß der folgenden Formel:

$$BMD = a + b \ R/H + d \ Xc/H,$$

   wobei a, b und d Gewichtungskoeffizienten sind; R der Widerstand der Person ist; Xc die Reaktanz der Person ist; und H die Größe der Person ist.

2. Bioimpedanz-Technologie-Verfahren zur Bewertung der lokalen oder Gesamt-Körper-Knochenmineraldichte (10) nach Anspruch 1, wobei in Schritt (a) das Verfahren für das Bioimpedanzmessgerät (20) zur Bestimmung des Widerstands und der Reaktanz der Person darin besteht, mehrere Elektrodensätze (21) des Bioimpedanzmessgeräts (20) zu bestimmen, wobei die mehreren Elektrodensätze (21) mit den Händen und Füßen, Händen oder Füßen der Person in Kontakt stehen, um eine Messschleife zu bilden, wobei jeder Elektrodensatz eine Messelektrode (23) und eine Stromelektrode (25) umfasst, wobei die Messelektrode (23) und die Stromelektrode (25) 1-10 cm voneinander beabstandet sind.

3. Bioimpedanz-Technologie-Verfahren zur Bewertung der lokalen oder Gesamt-Körper-Knochenmineraldichte (10) nach Anspruch 1, wobei in Schritt (a) die Person die Messung des Bioimpedanz-Messgeräts (20) wahlweise in Rückenlage, stehend oder sitzend durchführen soll.

**Revendications**

1. Méthode de la technologie de la bioimpédance pour évaluer la densité minérale osseuse locale ou du corps entier (10), comprenant les étapes de :

   (a) mesurer une résistance et une réactance d'un sujet à l'aide d'un instrument de mesure de la bioimpédance (20), et obtenir une taille dudit sujet ;
   (b) calculer la densité minérale osseuse du corps local ou entier dudit sujet à l'aide de l'instrument de mesure de la bioimpédance (20) selon la formule suivante :

$$DMO = a + b \ R/H + d \ Xc/H,$$

   où a, b et d sont des coefficients de pondération ; R est la résistance dudit sujet ; Xc est la réactance dudit sujet ; H est la taille dudit sujet.

2. Méthode de la technologie de la bioimpédance pour évaluer la densité minérale osseuse locale ou du corps entier (10) selon la revendication 1, dans laquelle à l'étape (a), la méthode pour que ledit instrument de mesure de la bioimpédance (20) mesure la résistance et la réactance dudit sujet consiste à mesurer plusieurs ensembles d'électrodes (21) dudit instrument de mesure de la bioimpédance (20), Ces ensembles d'électrodes multiples (21) sont en contact avec les mains et les pieds, les mains ou les pieds du sujet pour former une boucle de mesure, chaque ensemble d'électrodes comprenant une électrode de détection (23) et une électrode de courant (25), l'électrode de détection (23) et l'électrode de courant (25) étant distantes de 1 à 10 cm l'une de l'autre.

3. Méthode de la technologie de la bioimpédance pour évaluer la densité minérale osseuse locale ou du corps entier (10) selon la revendication 1, dans laquelle, à l'étape (a), ledit sujet doit effectuer la mesure de l'instrument de mesure de la bioimpédance (20) en position couchée, debout ou assise, au choix.

sitting position selectively.

EP 4 052 645 B1

10

(a)Measure the resistance and reactance of a subject by a bioimpedance measuring instrument, and obtain the height of the subject

(b)Calculate the bone mineral density of the local or whole body of the subject by the formula: BMD=a + b R/H+d Xc/H, where a, b, and d are weighting coefficients; R is the resistance of the subject; Xc is the reactance of the subject; H is the height of the subject.

FIG. 1

EP 4 052 645 B1

```
                        ┌──────────────────────────────────────┐ ⟋20
                        │  Bioimpedance measuring instrument   │
                        └──────────────────────────────────────┘
```

FIG. 2

| | Constant | Coefficient | | r | P (model) |
|---|---|---|---|---|---|
| | | Resistance/Height | Reactance/Height | | |
| $BMD_{Total}$ | 1.4421 | -0.0016[**] | 0.0051[*] | 0.659 | <0.0001 |
| $BMD_{Femur}$ | 1.0871 | -0.0025[**] | 0.0051[*] | 0.698 | <0.0001 |
| $BMD_{LS}$ | 1.3451 | -0.0015[**] | 0.0040[*] | 0.632 | <0.0001 |

[*], $P < 0.05$

[**], $P < 0.001$

FIG. 3

**EP 4 052 645 B1**